Europäisches Patentamt

European Patent Office

Office européen des brevets

(19)

(11) Publication number: **0 108 845**
**B1**

(12) **EUROPEAN PATENT SPECIFICATION**

(45) Date of publication of patent specification: **27.04.88**

(51) Int. Cl.⁴: **G 01 N 9/24**

(21) Application number: **82306026.4**

(22) Date of filing: **11.11.82**

(54) **Measuring device.**

(43) Date of publication of application:
**23.05.84 Bulletin 84/21**

(45) Publication of the grant of the patent:
**27.04.88 Bulletin 88/17**

(84) Designated Contracting States:
**AT BE CH DE FR GB IT LI LU NL SE**

(56) References cited:
**DE-A-1 498 746**
**DE-A-2 602 761**
**US-A-3 628 375**

(73) Proprietor: **SEAMAN NUCLEAR CORPORATION**
**7315 South First Street**
**Oak Creek Wisconsin 53154 (US)**

(72) Inventor: **Seaman, Donald J.**
**7315 South First Street**
**Oak Creek Wisconsin 53154 (US)**

(74) Representative: **Fisher, Bernard et al**
**Raworth, Moss & Cook 36 Sydenham Road**
**Croydon Surrey CR0 2EF (GB)**

## Description

This invention relates to a measuring device for measuring the density or moisture of a material.

In certain paving operations, such as, for example, when a layer of asphalt is applied to a pre-existing concrete roadway, the asphalt is commonly applied with a paver which lays the material at a pre-selected thickness. Typically, a roller is then employed for compacting the material. The actual density of the asphalt will be influenced by a number of factors, including the vibration frequency and amplitude of the compactor, ballast, the forward travel speed, the temperature of the material, the degree of overlap, the number of roller passes and roller travel speed, and the reaction surface to which the material is being applied. The thickness of the material being applied may also affect the efficiency of the compactor and therefore, influence density.

It is important to know the material density to prevent over-rolling. This occurs when the maximum density of the material has been attained and further compaction thereafter tends to loosen the material. Also, in the compaction of asphaltic concrete, it is important to know how close the compactor can approach the asphalt paver and how far behind it can traverse before chilling is encountered. If the compactor is too close to the paver, the hot material displaces and cannot be compacted. On the other hand, if the roller trails the compactor by too great a distance, the material will cool excessively at the far end of the traverse so that compaction in that area is also not possible.

It is, therefore, desirable to provide a method for rapidly and accurately determining the density of an applied layer while it was still hot so that corrections in the compacting operation can be made to eliminate the necessity for costly reworking.

DE—A—1498746 discloses a stationary meter of a type where the meter is mounted on a flat base. In order for proper operation, the base should be in planar contact with the surface of the material being measured. Such a device requires surface preparation to ensure a planar surface and cannot be used for making continuous measurements in close association with a compacting device.

US—A—3628375 discloses an ultra sonic thickness measuring gauge where the thickness is determined by measuring the time difference required for an ultra sonic wave to impact the upper and lower surfaces of the material being measured. By tilting a transducer, discontinuities in the surface of the material can be detected. The disclosed device is not sensitive to the angular orientation of the source or its distance from the material being measured. An ultra sonic source and a detector are disposed within a flexible tire located between end wheels. The purpose of the tire is to contain a coupling fluid, the position of the source relative to the material being achieved by end wheels. End portions of the tire are out of contact with the material and only the centre portion of the tire engages the material as a result of sag. Accordingly, the profile of the tire opposite the material is not a line but rather an arc and only a centre portion of the arc is in contact with the material.

According to the present invention, there is provided a measuring device for measuring the density or moisture of a material, the device having a shielded radioactive source and a radiation detector mounted on a support, characterised in that a first roller is mounted on the support and surrounds the source and detector and has a cylindrical outer surface for engaging the surface of the material whose density or moisture is being measured with line contact, the source and detector being mounted on said support a predetermined distance from the cylindrical surface and orientated radially relatively to the cylindrical surface, and a second roller coupled to the support and disposed laterally thereof for engaging the surface of the material being measured at a location spaced laterally of the line of contact between the first roller and the material, for supporting the source and the detector without reorientation relative to the surface of the material as the rollers are rolled over the material surface.

For a better understanding of the invention and to show how the same may be carried into effect, reference will now be made, by way of example, to the accompanying drawings, in which:—

Figure 1 shows one example of a mobile device in which the density measuring device may be mounted;

Figure 2 is a front elevational view, partly in section, of the density measuring device according to a preferred embodiment;

Figure 3 is a cross-sectional view taken along lines 3—3 of Figure 2;

Figure 4 is the top plan view of the density measuring device shown in Figure 2; and

Figure 5 is the side elevational view of the density measuring device shown in Figure 2.

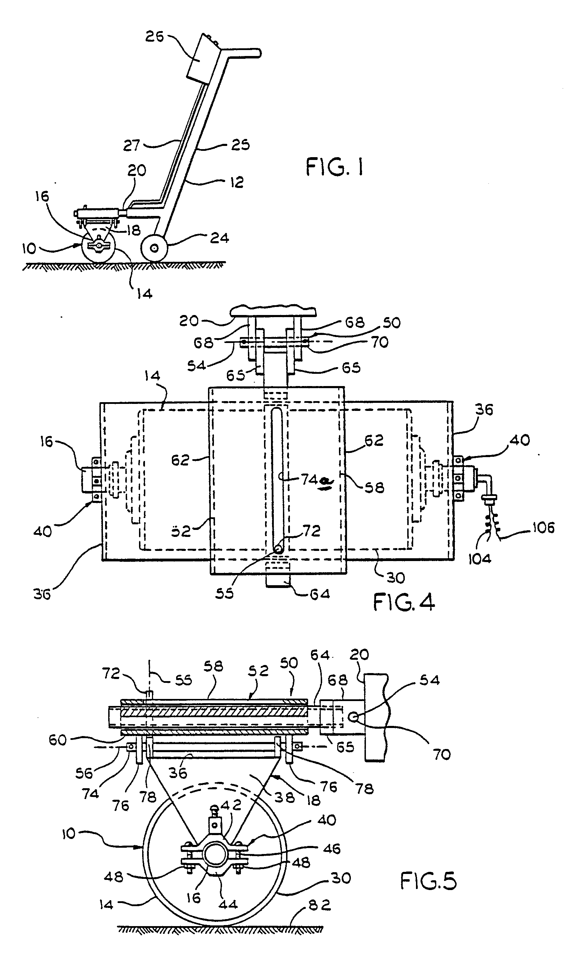

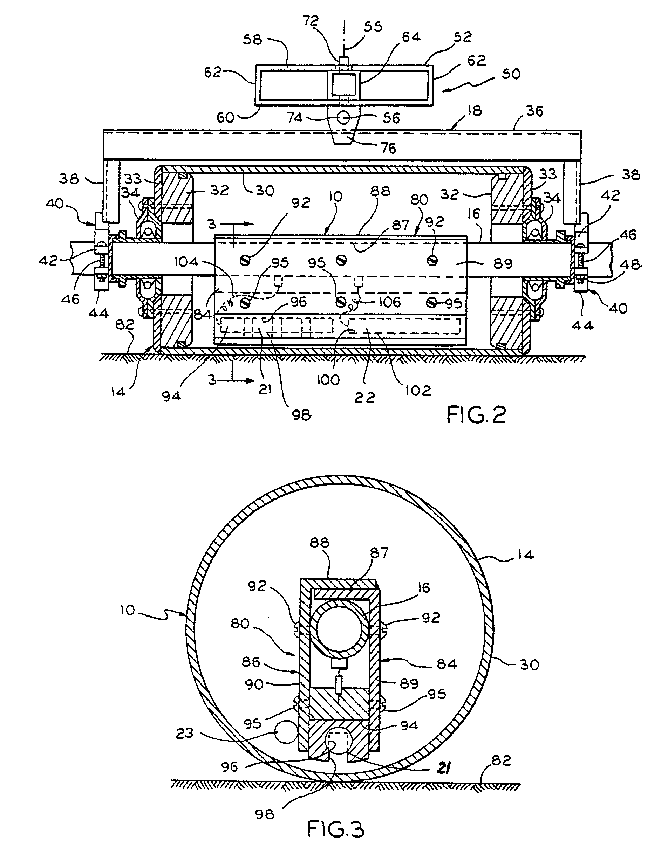

Figure 1 shows the measuring device 10 according to the preferred embodiment to be mounted on a manual transporter 12. In general terms, the measuring device includes a cylindrical roller 14 mounted for rotation on a shaft 16 which in turn is supported on a bracket assembly 18 carried adjacent the front end of the base 20 of transporter 12. A radiation source 21 and detectors 22 and 23 are supported within the roller 14 (see Figures 2, 3) as will be discussed more fully below. At the rear of the base 20 there are a pair of spaced apart wheels 24 and an upwardly extending handle 25. A microprocessor, a counter and a control panel 26 are mounted adjacent the upper end of handle 25 and are connected to the detectors 22 and 23 by conductors 27.

The roller 14 is shown more specifically in Figures 2 and 3 to comprise a hollow, cylindrical member 30 and a pair of relatively thick annular end members 32 which are suitably secured to the cylindrical member 30 and provide a stiffening thereto. There are also an annular end cap 33 secured to each member and each supports a suitable bearing 34 which rotatably support the roller 14 on the shaft 16. The shaft 16, the cylindrical member 30, the members 32 and the end caps 33 are preferably fabricated of the lightweight metallic material, such as aluminium. The surface of member 30 may also be coated with a material such as teflon® so as to minimize the tendency of asphalt and other materials to adhere to its surface.

As seen in Figures 2, 4 and 5, the bracket 18 includes a generally rectangular top plate 36 and a pair of depending triangular side plates 38. A set of quick release clamps 40 secures the shaft 16 to the lower end of side plates 38 and in general parallels them with the top plate 36. In particular, each clamp 40 includes a top, downwardly facing jaw member 42 affixed to the lower corner of its associated side plate 38 and engaging the upper portion of shaft 16 and an upwardly facing lower jaw member 44 which engages the lower portion of shaft 16. The members 42 and 44 are interconnected by a pair of screws 46 which extend through suitable aligned openings formed in the ends of each clamp member and secured by nuts 48. The clamps 40 permit the rapid release of the shaft 16 from the bracket 18 so that the roller 14 and it radioactive source can be safely stored when not in use.

The bracket 18 is mounted on the base 20 by means of a universal support assembly 50 which permits the roller 14 to pivot three dimensionally so that it may follow the contour of the surface being traversed. More particularly, the assembly 50 includes a frame 52 which is connected to the base 20 for pivotal movement about an axis 54 and which supports the bracket 38 for pivotal movement about axes 55 and 56 which are perpendicular to each other and to the axis 54.

The frame 52 comprises a pair of parallel spaced-apart plates 58 and 60 which are interconnected by side portions 62. A hollow, rectangular rod 64 is disposed between plates 58 and 60 has a pair of apertured members 65 affixed to one end and disposed between a pair of spaced-apart apertured bracket members 68 mounted at the front of the carriage base 20. A pin 70 extends through the apertures in the members 65 and the bracket 68 and defines the pivot axis 54. A second pin 72 extends upwardly from rod 64 at a point adjacent its opposite end and is received in a slot 74a formed in plate 58. The pin 72 defines the pivot axis 55. Finally, a third pin 74 which defines the pivot axis 56 extends through brackets 76 affixed in spaced-apart relation and extending downwardly from the plate 60. The pin 74 also extends through apertures formed-in-hinge members 78 affixed to the upper surface of plate 36.

Those skilled in the art will appreciate that the frame 52 and roller 14 supported thereby can pivot about the axis 54 of pin 70 while the frame and roller can swivel on pin 72 relative to rod 64 and that the roller 14 can pivot about axis 56 of pin 74 or relative to frame 52. In this manner, the roller 14 is free to follow the contour of the surface of the material being traversed. In addition, the pin 72 and slot 74a permits the frame 52 and roller 14 to be repositioned relative to the base 20 to facilitate forward or rearward rolling movement relative to surface 82.

As seen in Figures 2 and 3, the source 21 and the detectors 22 and 23 are mounted on shaft 16 by means of a support assembly 80 such that the source and detectors will remain a relatively fixed distance above the surface 82 of the material being measured as the roller 14 is rolled therealong. More specifically, the assembly 80 includes a pair of elongated support members 84 and 86 which are generally L-shaped in vertical section and are respectively defined by short legs 87 and 88 and long legs 89 and 90. The member 84 is affixed to shaft 60 with its short leg 86 engaging the upper-most portion of shaft 16 and leg 89 extending downwardly along one side and in engagement therewith. In addition, member 86 is mounted with its short leg 88 engaging the leg 87 and its long leg 90 extending downwardly along the opposite sides of shaft 16. A plurality of screws 92 affix the members 84 and 86 to the shaft 16 so that the legs 89 and 90 extend vertically downwardly in a generally parallel, spaced relation. Disposed in the gap between the lower ends of legs 89 and 90 is an elongate, generally rectangular in section, shield 94 which is formed of any suitable material such as lead. The shield 94 is suitably secured in this position in any suitable manner, such as by screws 95. The source 21 is disposed in a first cavity 96 formed in shield 94. An opening 98 in the lower end of cavity 96 exposes the surface 82 immediately below the source 21 to the radiation emitting from source 21 while the shield 94 prevents radiation upwardly and to the sides. The first detector 22 is similarly disposed in a recess 100 in shield 94 and there is a similar opening 102 in the lower end of the shield which permits reflected or scattered radiation from the source 21 to impact the detector 22. Detector 23 on the other hand is positioned on the leg 90 exteriorly shield 92 and adjacent to the source 21. Electrical conductors 104 and 106 are connected to detectors 22 and 23 respectively and extend therefrom to the interior of shaft 16 from which they emerge at one end for connection to the counter, microprocessor and control panel 26.

It will be appreciated that as the roller 14 is rolled across the surface of the material being measured, the source 21 and the detectors 22 and 23 will remain in a relatively fixed distance above the surface 82 of the material.

Those skilled in the art will appreciate that when a layer of asphalt, for example, is being applied to a base layer of another material, such as concrete, it is common to employ a device such as a paver, which is preset to apply material at predetermined thickness. To achieve the desired density, the asphaltic material must be compacted by making a number of passes over the asphalt layer with a heavy roller, for example. However, because of variables, such as temperature, ballasts, the surface to which the material is being applied, the frequency of vibration of the compactor and the number of roller passes, actual density of the material may vary. The deviation of any of these variables from optimums can substantially affect rolling times. The density measuring apparatus shown can be used in conjunction with the roller to measure the material density as the roller is traversing its surface so that rapid adjustments can be made to ensure compliance with specifications. In addition, adjustments in the variable factors can be made so as to minimize rolling times. Further, the present device permits the source 21 to be located relatively close to the surface 82 for safety and economy.

More specifically, the thickness of the top layer of asphalt material is first determined from the specifications, and that information is provided to the microprocessor 26. The microprocesor will also be provided with an instrument constant which is determined when the device is manufactured and which will depend upon the nature and strength of the source 21, the geometry of the apparatus and the sensitivity of the detectors 22 and 23 and will vary slightly from instrument to instrument.

Before the layer of material whose density is to be measured is applied, the density measuring device will be employed to provide an average density measurement for the sub-layer. This information will also be provided to the microprocessor for determination of density in a well-known manner. After the material laying apparatus has applied the surface layer and the same has been partially compacted by a roller, the manual transporter 12 will be passed over the surface and continuous reading of density will appear on the instrument panel 26 whereby the operator can immediately determine what further compacting or adjustments, if any, is required. Density is measured by the air-backscatter and will be calculated by the microprocessor in accordance with the method disclosed in US—A—4389136 filed January 23, 1981 and published June 21, 1983 and assigned to the assignee of the present invention. In addition, moisture may be measured in the conventional manner by the detector 23. For example, the method employed by the Seaman Nuclear Series 100 Density/Moisture Meter may be employed.

While only a single embodiment of the invention has been illustrated and described, it is not intended to be limited thereby. For example, instead of mounting the roller 14 on a manual transporter, the same could be mounted directly on a roller compacter and with the microprocessor, counter and instrument panel mounted in position for easy observation by the operator. In this manner, the operator of the roller could make direct readings and adjust his rolling passes accordingly.

## Claims

1. A measuring device for measuring the density or moisture of a material, the device having a shielded radioactive source (21) and a radiation detector (22, 23) mounted on a support (20), characterised in that a first roller (14) is mounted on the support and surrounds the source and detector and has a cylindrical outer surface (30) for engaging the surface of the material whose density or moisture is being measured with line contact, the source and detector being mounted on said support a predetermined distance from the cylindrical surface and orientated radially relatively to the cylindrical surface, and a second roller (24) coupled to the support and disposed laterally thereof for engaging the surface of the material being measured at a location spaced laterally of the line of contact between the first roller and the material, for supporting the source and the detector without reorientation relative to the surface of the material as the rollers are rolled over the material surface.

2. A measuring device according to claim 1, characterised in that the support (20) comprises a carriage.

3. A measuring device according to claim 2, characterised in that an indicator (26) is mounted on said carriage and connected to said detector (22, 23) for processing signals from said detector and for displaying an indication of density or moisture, said indicator (26) being mounted on said carriage on a position above the first roller in which the source and detector are disposed to permit observation by an operator.

4. A measuring device according to claim 1 or 2, characterised in that said second roller (24) comprises a roller compactor.

5. A measuring device according to claim 4, characterised by an indicator (26) mounted on said roller compactor and connected to said detector for processing signals from said detector and for displaying an indication of density or moisture, said indicator (26) being mounted on said compactor in a position above the first roller (14) in which the source and detector are disposed to permit observation by an operator.

6. A measuring device according to any one of the preceding claims, characterised in that a shaft (16) is fixed to said support, said first roller (14) being rotatably mounted on said shaft, said source and detector being mounted on said shaft and in a fixed relation relative thereto.

7. A measuring device according to claim 6, characterised in that a second support (84) extends downwardly from the shaft (16) and a shield (94) is fixed to the second support, and a first recess (96) is formed in the shield and has an opening (98) in its lower end, the source (21) being disposed in the first recess for directing radiation downwardly.

8. A measuring device according to claim 7, characterised in that a second recess (100) is formed in the shield and spaced from said first recess and opening downwardly, said detector (22) being disposed in the second recess for receiving radiation upwardly through said opening.

9. A measuring device according to claim 8, characterised in that the detector (23) is mounted on said second support and exteriorly of said second recess (100) but adjacent thereto.

10. A measuring device according to any one of claims 6 to 9 as appendant to claim 2, characterised in that a mounting means (50, 62, 64, 65, 68, 70) is provided for coupling the shaft to the firstmentioned support (20), said mounting means being constructed and arranged to permit limited universal pivotal movement of said shaft relative to the carriage.

11. A measuring device according to claim 10, characterised in that the mounting means includes a frame (52), a first coupling (65, 70) for connecting the frame to the support (20) for pivotal movement about a first axis, and a second coupling (72, 74) for connecting the frame to the shaft (16) for pivotal movement about a pair of axes which are perpendicular to each other and the first axis.

12. A measuring device according to claim 11, characterised in that the frame (52) is coupled to the firstmentioned support (20) for limited sliding movement in a direction generally parallel to said first axis.

**Patentansprüche**

1. Meßeinrichtung zum Messen der Dichte oder Feuchtigkeit eines Materials, wobei die Einrichtung eine abgeschirmte radioaktive Quelle (21) und einen Strahlungsdetektor (22, 23), angebracht auf einem Träger (20), hat, dadurch gekennzeichnet, daß eine erste Rolle (14) am Träger angebracht ist und die Quelle und den Detektor umgibt und eine zylindrische Außenfläche (30) zur Auflage auf der Fläche der Materials, dessen Dichte oder Feuchtigkeit gemessen wird, in Linienberührung hat, wobei die Quelle und der Detektor am Träger in einem vorherbestimmten Abstand von der zylindrischen Fläche angebracht und relativ zur zylindrischen Fläche radial ausgerichtet sind, und daß eine zweite Rolle (24) mit dem Träger gekuppelt und seitlich hievon, zur Auflage auf der Fläche des Materials, das gemessen wird, an einer Stelle in seitlichem Abstand von der Berührungslinie zwischen der ersten Rolle und dem Material, angeordnet ist, um die Quelle und dem Detektor ohne Neuausrichtung relativ zur Fläche des Materials abzustützen, wenn die Rollen über die Materialfläche gerollt werden.

2. Meßeinrichtung nach Anspruch 1, dadurch gekennzeichnet, daß der Träger (20) einen Wagen umfaßt.

3. Meßeinrichtung nach Anspruch 2, dadurch gekennzeichnet, daß ein Anzeigegerät (26) am Wagen angebracht und mit dem Detektor (22, 23) verbunden ist, um vom Detektor herrührende Signale zu verarbeiten und eine Anzeige von Dichte oder Feuchtigkeit darzustellen, wobei das Anzeigegerät (26) am Wagen in einer Position oberhalb der ersten Rolle, in der die Quelle und der Detektor angeordnet sind, angebracht ist, um eine Beobachtung durch eine Bedienungsperson zu ermöglichen.

4. Meßeinrichtung nach Anspruch 1 oder 2, dadurch gekennzeichnet, daß die zweite Rolle (24) einen Rollenverdichter umfaßt.

5. Meßeinrichtung nach Anspruch 4, gekennzeichnet durch ein Anzeigegerät (26), das am Rollenverdichter angebracht und mit dem Detektor verbunden ist, um vom Detektor herrührende Signale zu verarbeiten und eine Anzeige von Dichte oder Feuchtigkeit darzustellen, wobei das Anzeigegerät (26) am Verdichter in einer Position oberhalb der ersten Rolle (14), in der die Quelle und der Detektor angeordnet sind, angebracht ist, um eine Beobachtung durch eine Bedienungsperson zu ermöglichen.

6. Meßeinrichtung nach einem der vorhergehenden Ansprüche, dadurch gekennzeichnet, daß eine Achswelle (16) am Träger befestigt ist, wobei die erste Rolle (14) drehbar auf der Achswelle gelagert ist, die Quelle und der Detektor in fester Lage relativ zur Achswelle auf dieser angebracht sind.

7. Meßeinrichtung nach Anspruch 6, dadurch gekennzeichnet, daß sich ein zweiter Träger (84) von der Achswelle (16) abwärts erstreckt und ein Schirm (94) am zweiten Träger befestigt ist, und eine erste Ausnehmung (96) im Schirm gebildet ist und eine Öffnung (98) in ihrem unteren Ende hat, wobei die Quelle (21) in der ersten Ausnehmung angeordnet ist, um Strahlung abwärts zu richten.

8. Meßeinrichtung nach Anspruch 7, dadurch gekennzeichnet, daß eine zweite Ausnehmung (100) im Schirm gebildet und in Abstand von der ersten Ausnehmung vorgesehen ist und sich abwärts öffnet, wobei der Detektor (22) in der zweiten Ausnehmung angeordnet ist, um Strahlung von unten durch die Öffnung zu empfangen.

9. Meßeinrichtung nach Anspruch 8, dadurch gekennzeichnet, daß der Detektor (23) am zweiten Träger und außerhalb der zweiten Ausnehmung (100), jedoch benachbart hiezu, angebracht ist.

10. Meßeinrichtung nach einem der Ansprüche 6 bis 9, unter Rückbeziehung auf Anspruch 2, dadurch gekennzeichnet, daß eine Befestigungseinrichtung (50, 62, 64, 65, 68, 70) zur Verbindung der Achswelle mit dem ersterwähnten Träger (20) vorgesehen ist, wobei die Befestigungseinrichtung konstruiert und angeordnet ist, um eine begrenzte allseitige Schwenkbewegung der Achswelle relativ zum Wagen zu erlauben.

11. Meßeinrichtung nach Anspruch 10, dadurch gekennzeichnet, daß die Befestigungseinrichtung ein Gestell (52), eine erste Kupplung (65, 70) zum Verbinden des Gestells mit dem Träger (20) für eine Schwenkbewegung um eine erste Achse und eine zweite Kupplung (72, 74) zum Verbinden des Gestells mit der Achswelle (16) für eine Schwenkbewegung um ein Paar Achsen enthält, die senkrecht zueinander und zur ersten Achse sind.

12. Meßeinrichtung nach Anspruch 11, dadurch gekennzeichnet, daß das Gestell (52) mit dem ersterwähnten Träger (20) in einer Richtung allgemein parallel zur ersten Achse begrenzt verschiebbar gekuppelt ist.

## Revendications

1. Dispositif de mesure de la densité ou de l'humidité d'un matériau, ce dispositif comprenant une source radioactive (21) protégée par un écran et un détecteur (22, 23) de rayonnement, monté sur un support (20), caractérisé en ce qu'un premier rouleau (14) est monté sur le support et entoure la source et le détecteur et comporte une surface extérieure cylindrique (30) en contact avec la surface du matériau dont la densité ou l'humidité doivent être mesurées par contact en ligne, la source et le détecteur étant montés sur ledit support à une distance déterminée de la surface cylindrique et orientés dans le sens radial par rapport à la surface cylindrique, et une deuxième rouleau (24) couplé au support et disposé à son côté en contact avec la surface du matériau à mesurer à un emplacement situé latéralement à une certaine distance de la ligne de contact entre le premier rouleau et le matériau, pour supporter la source et le détecteur sans réorientation par rapport à la surface du matériau quand les rouleaux passent sur la surface du matériau.

2. Dispositif de mesure selon la revendication 1, caractérisé en ce que le support (20) comprend un chariot.

3. Dispositif de mesure selon la revendication 2, caractérisé en ce qu'un indicateur (26) est monté sur ledit chariot et relié au dit détecteur (22, 23) pour traiter des signaux provenant dudit détecteur et pour afficher une valeur de densité ou d'humidité, ledit indicateur (26) étant monté sur ledit chariot à un emplacement situé au-dessus du premier rouleau où sont placés la source et le détecteur pour permettre l'observation par l'opérateur.

4. Dispositif de mesure selon l'une des revendication 1 ou 2, caractérisé en ce que ledit deuxième rouleau (24) comprend un appareil de compactage à rouleau.

5. Dispositif de mesure selon la revendication 4, caractérisé en ce qu'il comprend un indicateur (26) monté sur ledit appareil de compactage à rouleau et relié au dit détecteur pour traiter des signaux provenant de ce détecteur et pour afficher une valeur de densité ou d'humidité, cet indicateur (26) étant monté sur l'appareil de compactage à un emplacement situé au-dessus du premier rouleau (14) où sont placés la source et le détecteur pour permettre l'observation par l'opérateur.

6. Dispositif de mesure selon l'une des revendications 1 à 5, caractérisé en ce qu'un arbre (16) est fixé au dit support, le premier rouleau (14) étant monté de manière à pouvoir tourner sur ledit arbre, ladite source et le détecteur étant montés sur ledit arbre et dans une position fixe par rapport à lui.

7. Dispositif de mesure selon la revendication 6, caractérisé en ce qu'un deuxième support (84) descend de l'arbre (16) et qu'un écran protecteur (94) est fixé au deuxième support et une première partie rentrante (96) est ménagée dans l'écran et comporte une ouverture (98) dans son extrémité inférieure, la source (21) étant placée dans la première partie rentrante pour que le rayonnement soit dirigé vers le bas.

8. Dispositif de mesure selon la revendication 7, caractérisé en ce qu'une deuxième partie rentrante (100) est ménagée dans l'écran protecteur et située à une certain distance de ladite première partie rentrante et s'ouvre vers le bas, le détecteur (22) étant placé dans la deuxième partie rentrante pour recevoir le rayonnement dirigé vers le haut et traversant ladite ouverture.

9. Dispositif de mesure selon la revendication 8, caractérisé en ce que le détecteur (23) est monté sur le deuxième support et à l'extérieur de la deuxième partie rentrante (100), mais à proximité d'elle.

10. Dispositif de mesure selon l'une des revendications 6 à 9 dépendant de la revendication 2, caractérisé en ce qu'il comporte un dispositif de montage (50, 62, 64, 65, 68, 70) assurant le couplage de l'arbre avec le support (20) mentionné en premier lieu, ledit dispositif de montage étant réalisé et disposé de manière à permettre un mouvement de pivotement universel limité dudit arbre par rapport au chariot.

11. Dispositif de mesure selon la revendication 10, caractérisé en ce que le dispositif de montage comprend un cadre (52), un premier accouplement (65, 70) pour relier le cadre au support (20) par un mouvement de pivotement autour d'un premier axe et un deuxième accouplement (72, 74) pour relier le cadre à l'arbre (16) pour un mouvement de pivotement autour d'une paire d'axes qui sont perpendiculaires l'un à l'autre et au premier axe.

12. Dispositif de mesure selon la revendication 11, caractérisé en ce que le cadre (52) est couplé au support (20) mentionné en premier lieu pour un mouvement de glissement limité dans une direction sensiblement parallèle au dit premier axe.

0 108 845

FIG. 1

FIG. 4

FIG. 5

FIG.2

FIG.3